# EUROPEAN PATENT SPECIFICATION

(11) **EP 1 292 672 B1**
(45) Date of publication and mention of the grant of the patent: **29.03.2006**
(21) Application number: 01960337.2
(22) Date of filing: 18.06.2001
(51) Int. Cl.: C12N 11/04, C12C 11/09

(54) **IMMOBILIZING CARRIER COMPRISING A POROUS MEDIUM**
IMMOBILISIERUNGSTRÄGER MIT EINEM PORÖSEN MEDIUM
SUPPORT D'IMMOBILISATION COMPRENANT UN MILIEU POREUX

(30) Priority: 19.06.2000 EP 00202115
(43) Date of publication of application: 19.03.2003
(73) Proprietor: N.V. BEKAERT S.A., 8550 Zwevegem (BE); K.U. LEUVEN RESEARCH & DEVELOPMENT VZW, B-3000 Leuven (BE)
(72) Inventor: ARNAUT, Marc, B-9910 Knesselare (BE); ANDRIES, Marc, B-3010 Kessel-Lo (BE); DELVAUX, Freddy, B-3010 Kessel-Lo (BE); KELGTERMANS, Marc, B-3020 Herent (BE)
(86) International application number: PCT/EP2001/006939
(87) International publication number: WO 2001/098477

(56) References cited:
- WO-A-00/43337
- WO-A-00/49140
- WO-A-98/33882
- WO-A-99/07467
- US-A- 5 366 719
- DATABASE BIOSIS [Online] BIOSCIENCES INFORMATION SERVICE, PHILADELPHIA, PA, US; N. ROUHANA ET AL.,: "Development of a mebrane-based vapor-phase bioreactor" XP002152205
- DATABASE FOOD SCI. & TECH. ABS [Online] FSTA IFIS Publishing; M. ANDRIES ET AL.,: "Design of a multi-purpose immobilized yeast bioreactor system for application in the brewing process" XP002152206

## Description

### Field of the invention.

The invention relates to the use of a cell immobilising carrier in a novel continuous fermentation process. More particularly, this fermentation process is used in the continuous production of beer.

### Background of the invention.

lmmobilisation of fermenting cells is a technique that can be applied in brewing industries. It requires the retention of catalytic cells within a fermenter and their supply with nutrients.

In order to obtain an efficient system with a high productivity and in order to assure the quality of the final product, a number of requirements should be met.
First of all, the carrier should have a high mechanical strength, it should be chemically inert and should not be toxic to the cells.
Furthermore, a high loading capacity should be reached. The loading capacity is defined as the weight of cells to the surface of the carrier. In addition, the carrier should not have a negative effect on the supply of nutrients to the immobilized cells.
Furthermore, the system should be efficient, easy to operate and should give high yields.

Either due to the limited loading capacity and/or the diffusional limitations of the known carriers none of them have been successfully implemented in industrial brewing installations,
The inclusion or encapsulation of yeast cells in a polymeric gel, such as calcium alginate or κ-carrageenan beads has the drawback that the process is unstable for example because of an uncontrolled release of cells by dissolving of the polymer.
Other immobilising carriers are based on the attraction of negatively charged yeast cells by the positively charged groups of the carrier such as the DEAE groups of modified cellulose. However, the use of DEAE is dependent on packed beds, which are difficult to use in the main fermentation, because of diffusion limitations and channeling by CO₂, Another drawback of methods based on such a carrier is that other negatively charged groups such as proteins are also attracted and have to be removed in advance for example by precipitation.
A more recent group of immobilising carriers is based on the colonisation of yeast on a porous medium such as porous glass beads or ceramic material such as silicon carbide. However, their use requires circulation pumps or large amounts of CO₂ sparging in the fermentation unit.

### Summary of the invention.

It is an object of the present invention to provide the use of cell immobilising carrier with a high mechanical strength, which is chemically inert, easy to manipulate and which can easily be cleaned, which allows rapid colonisation and efficient CO₂ venting, with a high specific surface, which is therefore characterised by a high loading capacity, in fermentation processes, and more particularly in the continuous production of beer.

According to a first aspect of the invention the use of a cell immobilising carrier is provided. The carrier is in particular suitable for the immobilisation of fermenting cells such as yeast.
The immobilising carrier comprises a porous medium having a porosity which is higher than 70 %. More preferably, the porosity of the carrier is higher than 75 % and most preferably even higher than 80 %, for example 85 %.

A carrier characterised by such a high porosity can be obtained by making at least one layer of fibers, such as polymeric fibers or metal fibers. Possibly, the carrier comprises powder particles in addition to the fibers.

In order to avoid fiber migration, it is preferred that a certain degree of bonding between the fibers is obtained.

Techniques to provide webs of polymeric fibers are for example spun-bonding, spun-laying or melt blowing.

The carrier comprises at least one layer of metal fibers which has been sintered. Preferably, the medium is compacted.
Possibly, the carrier is a layered structure comprising a number of layers. Each of these layers comprises a web of metal fibers.
The different layers are stacked up to form a layered structure. The thus obtained layered structure is then sintered and compacted.

The diameter of the metal fibers is between 1 µm and 30 µm, for example between 22 and 30 µm.
The different webs of the layered structure can be made of fibers the same or having a different fiber diameter.

Metal fibers used for the immobilising carrier according to the invention may be made of conventional metal or metal alloys.
Preferred alloys are stainless steel such as stainless steel 316L, Hastelloy®, Inconel®, Nichrome®, Alloy HR.

These materials are inert, neutral in taste and food approved. They are characterised by a high mechanical strength and a high chemical resistance.

The compacting is preferably done by a cold isostatic pressing operation, since this allows obtaining a medium with a homogeneous pore size distribution over the entire surface.

It can be preferred to maximize the available surface of the porous medium into a minimum volume of the fermenter.
By maximising the available surface, the loading capacity is maximised. Preferably, the loading capacity is higher than 100 g/m² and more preferably higher than 150 g/m² for instance higher than 200 g/m².
A high loading capacity results in higher reaction rates and in an improved production yield.

As mentioned above, the porosity of the carrier is preferably high. The open pored structure makes an efficient CO₂ venting possible and allows rapid colonisation of the cells. An efficient CO₂ venting is an absolute requirement to obtain an optimal transport of material.
The high porosity of the material of the invention is a major advantage over the existing carriers, such as SiC carriers, which are hindered by diffusional limitations. These limitations result in a negative impact on the yeast performance.
Since the pores are homogeneously distributed over the entire surface, the fermentation process occurs homogeneously.
Furthermore, because of the high porosity of the carrier, the loading capacity is high.

The carrier used according to the invention has the advantage that it can be designed for optimal mass transfer.

Preferably, the carrier has an open structure with a high available surface.

Different embodiments can be considered.
The carrier may have a tubular form or alternatively it may comprise several tubes of the material according to the present invention, placed concentric around each other.
The medium can be folded, undulated, pleated or rippled before a tube is formed.
Possibly, a spacer layer may be provided between two consecutive concentric tubes. A suitable spacer layer is a mesh, for example a regular woven structure comprising metal wires. Also this spacer layer can be folded, undulated, pleated or rippled.

Alternatively, a number of concentric tubes comprising a mesh can be placed around each other and in-between these concentric tubes the medium according to the present invention, either undulated or not, can be disposed.

In a preferred embodiment, a web comprising metal fibers is rolled up along its longitudinal axis to form a wrap-around tube.
Also in this embodiment it can be advisable to make use of a spacer layer. A layered structure comprising the carrier and the spacer layer is thereby rolled up.
Either the web of metal fibers or the spacer layer can be folded, undulated, pleated or rippled.

A cell immobilising carrier used according to the present invention features the advantage that it can easily be scaled up by modular assembly.

A further advantage of the immobilising carrier is that it can easily be cleaned, for example by back-flushing. Since repeated back-flushing is allowed, the carriers have long life times.
The medium can be sterilised in a chemical way or by steam sterilisation.

The carrier is used as immobilising carrier for the production of fermented liquids. In particular it is intended to be used for the production of beer either with low or high fermentation. However, it can also be used for the production of other alcoholic beverages.
The ultimate objective is to use the carrier in the continuous production of beer.

According to a second aspect, the use of a cell immobilising carrier according to the present invention for the fermentation of yeast is provided. The immobilising carrier is in particular suitable to be used in a continuous yeast fermentation process.

An installation for the continuous production of alcoholic beverages is described.
The installation comprises a bioreactor, which comprises at least one immobilising carrier according to the present invention.
Preferably, this bioreactor is a gas lift draft tube bioreactor.

The immobilising carrier is located in the draft tube within the reactor.
Preferably, the carrier is placed in the lower part of the draft tube, more preferably the carrier is placed at the bottom of the draft tube.

Immobilisation of the fermenting cells is necessary to prevent wash out of the cells during continuous fermentation.
Preferably, more than 50% of the cells in the reactor are immobilised. This leads to a higher concentration of cells in the draft tube than in the annulus surrounding the draft tube, resulting in a higher CO₂ production in the draft tube.
Furthermore, due to the sintered metal fibers in the immobilising carrier additional CO₂ gas is produced in the draft tube, as CO₂ bubbles easily arise on sharp edges. In this way the concentration of soluble CO₂ is lowered which is beneficial to the fermentation process.

The higher production of CO₂ gas in the draft tube than in the annulus promotes the circulation of the fermenting medium within the reactor.

Due to its open structure with a high porosity, the immobilising carrier is especially useful in the gas lift draft tube bioreactor according to the present invention as the immobilising carrier is of little resistance to the circulating medium.
Within the installation the use of mechanical pumps or the sparging of large amounts of gas is not necessary to obtain a good mixing and circulation of the fermenting medium.
On the other hand, the mixing and circulation of the fermenting medium is driven by the CO₂ produced by the yeast cells.
Consequently, the installation according to the present invention can be considered as an energy-efficient installation.

The circulation of the fermenting medium favorises a stable continuous fermentation. Possibly, the movement of the fermenting medium can be optimised by limited additional sparging.

The immobilising carrier is especially useful in the primary fermentation step.

Since in such an installation the use of pumps is not necessary any more, the risk of contamination is diminished.

This risk of contamination is of particular importance in a continuous fermentation process.

According to a third aspect of the invention a method for the continuous production of beer or other alcoholic beverages by means of a fermentation process is provided. The fermentation process is carried out in a bioreactor, such as a gas lift draft tube bioreactor with an immobilising carrier as described above

A preferred method comprises the following steps :
- providing an immobilising carrier as described above;
- immobilising yeast on said carrier;
- supplying nutrient to the said bioreactor, preferably in a continuous way.
- transferring an amount of beer, preferably in a continuous way. The transferred beer can further be treated, the beer is for example transferred to the finishing tank for a finishing treatment.

### Brief description of the drawings.

The invention will now be described into more detail with reference to the accompanying drawings wherein
- FIGURE 1 to 6 show the cross-sections of different embodiments of immobilising carriers according to the invention.
- FIGURE 7 shows the installation for continuous fermentation.

### Description of the preferred embodiments of the invention.

A possible porous medium for an immobilising carrier comprises two layers, each of these layers comprises a web of metal fibers.
A first layer comprises a non-woven web of metal fibers with a diameter of 22 µm. The second layer comprises a web of metal fibers with a diameter of 30 µm.

Metal fibers are obtained by means of bundled drawing as for example described in US 3,379,000. The first and second layer are brought into contact with each other and in a subsequent step the obtained layered structure is sintered and compacted by a cold isostatic pressing operation.
The immobisiling carrier has a weight of 1200 g/m² and a porosity of 76.78 %.

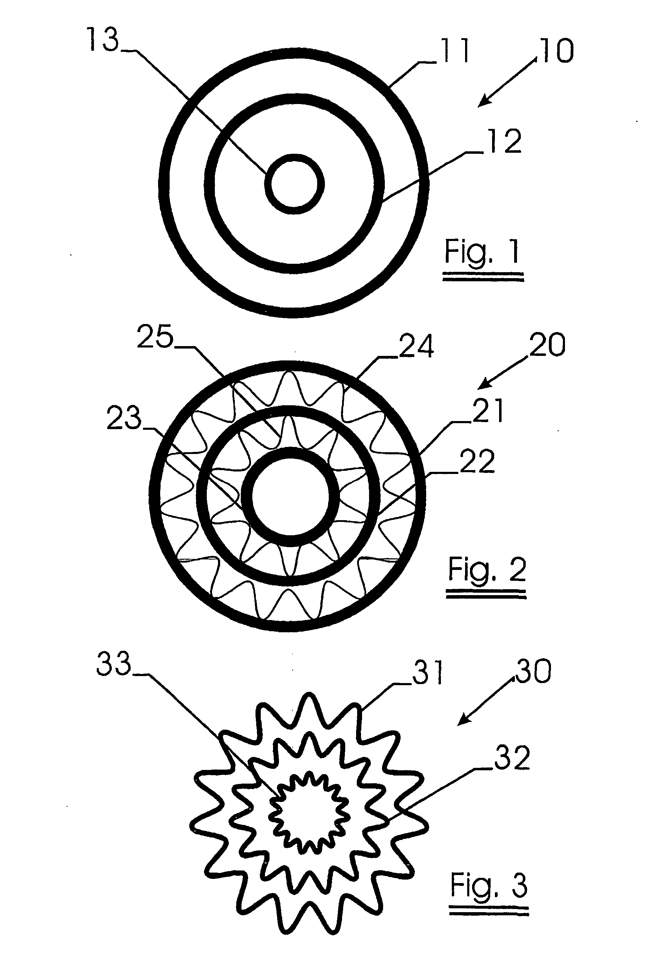

Different embodiments made from this porous medium can be considered. In principle, each carrier design comprising a medium with a high porosity and characterised by a high loading capacity can be used in the production of alcoholic beverages. In figures 1 to 6 some illustrative examples are shown.

Referring to figure 1, an immobilising carrier 10 comprises 3 concentric, spaced tubes 11, 12, 13.
The porous medium is rolled into the shape of a tube 11, 12, 13 and is welded. A number of these tubes can be placed concentric around each other to form the immobilising carrier.
Preferably, the tubes have a circular or elliptical cross-section.
The carrier has for example a length of 1500 mm and an outer diameter of 100 mm.
The size and number of the tubes determine the available surface of the carrier and as a direct influence on the loading capacity of the carrier.

Optionally, a spacer layer is interposed between two adjacent concentric tubes.
The cross-section of a preferred embodiment of an immobilising carrier 20, comprising three concentric tubes 21, 22, 23 and spacer layers 24, 25 inbetween two adjacent tubes, is shown in figure 2.
The spacer layer is for example a regular woven mesh, folded in the space created between two adjacent tubes.

Figure 3 shows the cross-section of a further embodiment. The immobilising carrier 30 comprises three concentric, spaced tubes 31, 32, 33.
The medium is undulated before the tubes are formed.
Possibly, a spacer layer can be disposed between two adjacent concentric tubes.

By wrapping the medium the available surface per volume unit can be increased.

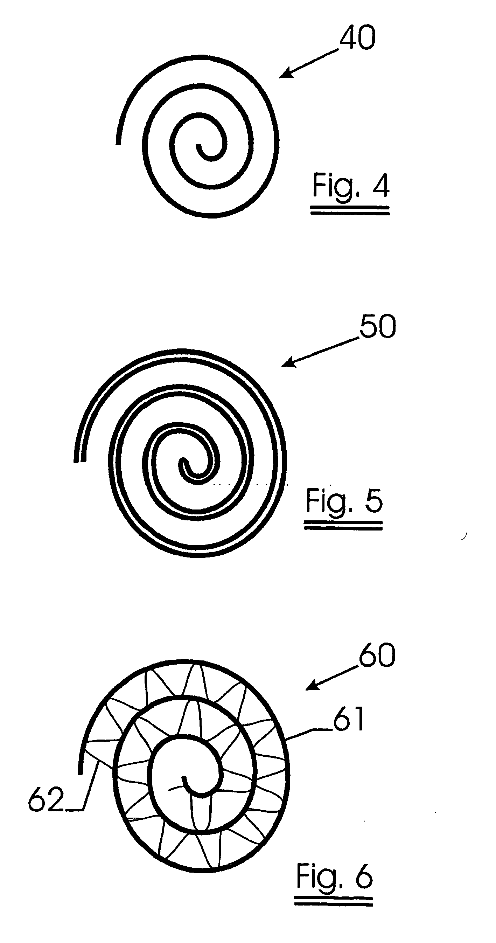

Figure 4 shows an embodiment in which a web of metal fibers with a porosity of 85 % is rolled up along its longitudinal axis to form a wrap-around tube. The cross-section of the carrier is a spiral curve.
The wrap-around tube has a length of 1500 mm and an outer diameter of 100 mm.

The porous medium can be folded, undulated, pleated or rippled before being rolled up.

Figure 5 shows an alternative way of wrapping the porous medium in order to increase the surface per volume unit.

The embodiment of figure 6 is obtained by
- forming a layered structure by bringing the porous medium 61 in contact with a spacer layer 62;
- rolling the layered structure to form a wrap-around tube.
   The spacer layer 62 is for example a folded mesh.

Alternatively, the mesh can be rolled up to form a wrap-around tube and the porous medium can be folded in the space created by the mesh.

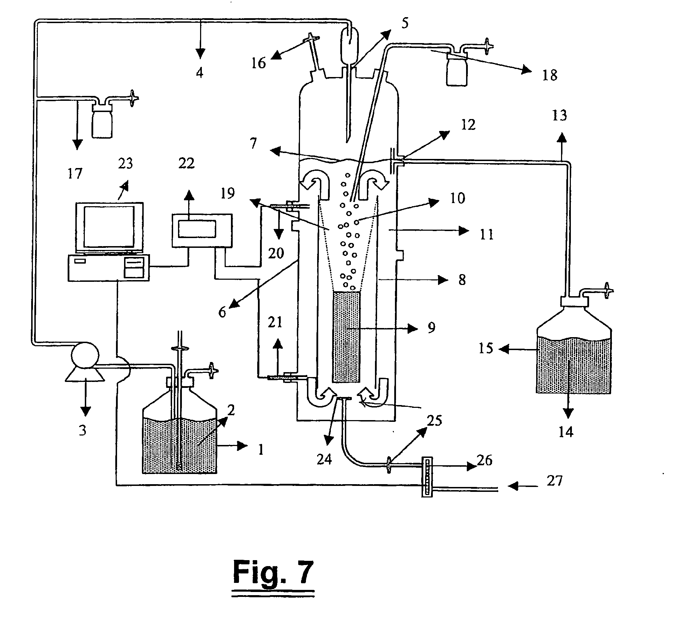

Figure 7 shows the installation comprising a feeding tank 1, a gas lift bioreactor 6 and a finishing tank 15.

A feeding pump 3 supplies fresh medium 2 to the said bioreactor 6 through the feeding pipe 4 and inlet 5. This medium mixes with the fermenting medium 7. The fermenting medium is moving downwards in the annulus 11 and moving upwards in the draft tube 8. This circulation is driven by the CO₂ gas 10 released from the immobilising carrier 9, which results in the formation of a density difference between the riser zone determined by the draft tube 8 and the downcomer zone determined by the annulus 11 surrounding the draft tube.

Two conductivity meters 20 and 21 measure the homogeneity of the fermenting medium 7. The two conductivity meters are connected to a reading device 22 that is connected to a computer 23.
The system is driven by the CO₂ produced by the yeast cells.
Normally, sparging or pumping is not necessary.

If a situation of not homogeneity occurs, the computer 23 will regulate an air valve 26 in such a way that an amount of gas is sparged as is necessary for optimal circulation.
For this purpose, the computer 23 is connected to an adjustable air valve 26. The air valve is placed between a gas-input line 27, for example for CO₂ and/or O₂, and a sparger 24. The sparger 24 is equipped with a sterile air filter 25.

The medium 2 is fermented by free cells in the fermenting medium 7 and by immobilised cells on the said carrier 9.
Preferably, the medium 2 is oxygenated to supply the fermenting cells in the said bioreactor with sufficient oxygen.
The fermented beer flows past outlet 12 through piping 13 in the finished beer vessel 14 wherein the finished beer 15 is collected.

The produced CO₂ can escape through a vent 16. It is possible to recover the produced CO₂ by a CO₂ recovery installation.

The said carrier 9 is fixed in the reactor by wires 19. The said carrier 9 is fixed at the bottom of the draft tube 8. The draft tube 8 is for example placed at 14% of the liquid height 7 within the said reactor 6. The relation reactor diameter / draft tube diameter equals the square root of 2. The height of the draft tube equals for example 75% of the liquid height. The liquid height equals for example 66% of the reactor height, the 34% of headspace is necessary for foam formation.

The immobilised cells can be removed by backflusing of the carrier.
Cold or hot water, NaOH solution and/or enzymatic solutions, such as glucanase and xylanase can thereby be used.
Sterilisation of the medium is possible, for example by steam sterilisation or by rinsing the medium with a mixture of peracetic acid and H₂O₂ (1.5 % v/v).

## Claims

1. The use of a cell immobilising carrier for the production of fermented liquids, said immobilising carrier comprising a porous medium, said porous medium comprising at least one layer of steel fibers which have been sintered, said steel fibers having a diameter between 1 µm and 30 µm and said medium having a porosity of at least 70 %.

2. The use according to claim 1, whereby said medium has a loading capacity of at least 100 g/m².

3. The use according to claim 1 or 2, whereby said carrier comprises a number of tubes made of said medium, said tubes are placed concentric around each other.

4. The use according to claim 3, whereby said carrier comprises a spacer placed between two consecutive concentric tubes.

5. The use according to any one of the preceding claims, whereby said medium is rolled up along its longitudinal axis to form a wrap-around tube.

6. A method for the continuous production of fermented liquids by means of a fermentation process on a cell immobilising carrier comprising a porous medium, said porous medium comprising at least one layer of steel fibers which have been sintered, said steel fibers having a diameter between 1 µm and 30 µm and said medium having a porosity of at least 70 % said method comprising the steps of
- providing the said cell immobilising carrier in a bioreactor;
- immobilising yeast on said carrier;
- supplying nutrient to said bioreactor;
- transferring an amount of fermented liquid preferably in a continuous way.

## Patentansprüche

1. Benutzung eines Zellimmobilisierungsträgers zur Herstellung vergorener Flüssigkeiten, wobei der Immobilisierungsträger ein poröses Mittel aufweist, das poröse Mittel mindestens eine Schicht aus Stahlfasern aufweist, die gesintert wurden, die Stahlfasern einen Durchmesser zwischen 1 µm und 30 µm aufweisen und das Mittel eine Porosität von mindestens 70 % aufweist.

2. Benutzung nach Anspruch 1, wobei das Mittel eine Beladungsfähigkeit von mindestens 100 g/m² aufweist.

3. Benutzung nach Anspruch 1 oder 2, wobei der Träger eine Anzahl von Rohren aufweist, die aus dem Mittel hergestellt sind, und die Rohre konzentrisch umeinander angeordnet sind.

4. Benutzung nach Anspruch 3, wobei der Träger einen Abstandhalter aufweist, der zwischen zwei aufeinanderfolgenden konzentrischen Rohren angeordnet ist.

5. Benutzung nach einem der vorhergehenden Ansprüche,
wobei das Mittel unter Bildung eines Wickelrohres um seine Längsachse aufgerollt wird.

6. Verfahren zur kontinuierlichen Herstellung vergorener Flüssigkeiten mittels eines Gärverfahrens an einem Zellimmobilisierungsträger, der ein poröses Mittel aufweist, wobei das poröse .Mittel mindestens eine Schicht aus Stahlfasern aufweist, die gesintert wurden, die Stahlfasern einen Durchmesser zwischen 1 µm und 30 µm aufweisen, das Mittel eine Porosität von mindestens 70 % aufweist und das Verfahren die folgenden Schritte aufweist:
- Bereitstellen, des Zellimmobilisierungsträgers in einem Bioreaktor,
- Immobilisieren von Hefe auf dem Träger,
- Versorgen des Bioreaktors mit Nährstoff,
- Überführen einer Menge an vergorener Flüssigkeit, vorzugsweise in einer kontinuierlichen Weise.

## Revendications

1. Utilisation d'un support d'immobilisation de cellules pour la production de liquides fermentés, ledit support d'immobilisation comprenant un milieu poreux, ledit milieu poreux comprenant au moins une couche de fibres d'acier qui ont été frittées, lesdites fibres d'acier présentant un diamètre entre 1 µm et 30 µm et ledit milieu ayant une porosité d'au moins 70%.

2. Utilisation selon la revendication 1, par laquelle ledit milieu a une capacité de charge d'au moins 100 g/m².

3. Utilisation selon la revendication 1 ou 2, par laquelle ledit support comprend un nombre de tubes faits dudit milieu, lesdits tubes sont placés de manière concentrique les uns autour des autres.

4. Utilisation selon la revendication 3, par laquelle ledit support comprend un espaceur placé entre deux tubes concentriques consécutifs.

5. Utilisation selon l'une quelconque des revendications précédentes, par laquelle ledit milieu est roulé le long de son axe longitudinal pour former un tube enveloppant.

6. Procédé de production en continu de liquides fermentés au moyen d'un processus de fermentation sur un support d'immobilisation de cellules comprenant un milieu poreux, ledit milieu poreux comprenant au moins une couche de fibres d'acier qui ont été frittées, lesdites fibres d'acier présentant un diamètre entre 1 µm et 30 µm, et ledit milieu ayant une porosité d'au moins 70%, ledit procédé comprenant les étapes suivantes :
- fournir ledit support d'immobilisation de cellules dans un bioréacteur;
- immobiliser la levure sur ledit support;
- fournir une substance nutritive audit bioréacteur, et
- transférer une quantité de liquide fermenté, de préférence d'une façon continue.
